# FASCICULE DE BREVET EUROPEEN

(11) **EP 0 904 061 B1**
(45) Date de publication et mention de la délivrance du brevet: **09.10.2002**
(21) Numéro de dépôt: 97952996.3
(22) Date de dépôt: 23.12.1997
(51) Int. Cl.: A61K 9/14

(54) **COMPOSITION PHARMACEUTIQUE A BASE DE RHEINE OU DE DIACERHEINE A BIODISPONIBILITE AMELIOREE**
ARZNEIZUBEREITUNG BESTEHEND AUS RHEIN ODER DIACERHEIN MIT VERBESSERTER BIOVERFÜGBARKEIT
PHARMACEUTICAL COMPOSITION WITH BASE OF RHEIN OR DIACERHEIN WITH IMPROVED BIOLOGICAL AVAILABILITY

(30) Priorité: 23.12.1996 FR 9615867
(43) Date de publication de la demande: 31.03.1999
(73) Titulaire: Mazal Pharmaceutique, 29000 Quimper (FR)
(72) Inventeur: ESTANOVE, Cyril, F-92100 Boulogne (FR); PRUDHOMME, Alain, F-29940 La Forêt Fouesnant (FR)
(74) Mandataire: L'Helgoualch, Jean
(86) Numéro de dépôt international: FR9702403
(87) Numéro de publication internationale: WO98027965

(56) Documents cités:
- EP-A- 0 330 532
- EP-A- 0 371 431
- EP-A- 0 446 753
- WO-A-94/14421

## Description

La présente invention concerne une nouvelle composition pharmaceutique stable utilisable en thérapeutique humaine et vétérinaire, et plus particulièrement une nouvelle forme galénique adaptée à la rhéine et à la diacétylrhéine (diacérhéine), améliorant de manière sensible la biodisponibilité du principe actif.

La rhéine, ou acide 9,10-dihydro-4,5-dihydroxy-9,10-dioxo-2-anthracène carboxylique, et la diacérhéine, son dérivé diacétylé, c'est-à-dire l'acide 4,5-bis(acétyloxy)-9,10-dihydro-4,5-dihydroxy-9,10-dioxo-2-anthracène carboxylique, sont décrites dans de nombreuses publications scientifiques ainsi que dans le brevet FR-A-2.508.798. La diacérhéine est bien connue comme médicament présentant une activité antiarthrosique, et est utilisée en particulier dans le traitement de l'arthrose. Cependant, la rhéine et la diacérhéine présentent l'inconvénient d'être pratiquement insolubles dans l'eau et dans les alcools. De plus, la diacérhéine peut être administrée par voie orale, mais elle n'est pas totalement absorbée par le tube digestif, et cette absorption incomplète pourrait entraîner des effets secondaires indésirables, par exemple des effets laxatifs. D'autre part, l'administration de la rhéine poserait encore plus de problèmes que celle de la diacérhéine.

Pour surmonter ces difficultés, on a proposé dans la littérature divers dérivés, ainsi que des compositions pharmaceutiques et des formes galéniques spécifiques. Par exemple, le brevet EP-A-243.968 décrit un sel de potassium de diacérhéine soluble dans l'eau, qui peut être utilisé dans la préparation de compositions pour administration parentérale.

On sait d'autre part que la solubilisation et/ou la mouillabilité d'une substance peuvent être améliorées par traitement au moyen d'un agent tensio-actif, ce qui a généralement pour effet de favoriser la biodisponibilité du principe actif. On sait également que le broyage de principes actifs insolubles en présence de certains polymères hydrosolubles améliore la solubilité et la biodisponibilité du produit (Yamamoto et al., J. Pharm. Sci. (1976) 65, p. 1484-88).

Le broyage des substances utilisées en thérapeutique peut être réalisé dans des broyeurs à boulets ou à marteaux de type usuel. On peut également procéder par micronisation dans des microniseurs à jet gazeux qui présentent l'avantage de ne pas échauffer les substances à microniser. La technique de la micronisation a été utilisée pour mettre au point une composition pharmaceutique à base de progestérone biodisponible et administrable par voie orale, dont la biodisponibilité est le double de celle de la forme cristallisée antérieurement connue.

Le brevet EP-A-330.532 décrit la comicronisation du fénofibrate en présence de laurylsulfate de sodium. Par contre, M. Otsuda et al. (J.P.S. 84 (1995) p. 1434-37) ont étudié la micronisation de la phénitoine en présence d'un agent tensio-actif, et ils ont montré que la solubilité de la phénitoine n'est pas améliorée dans le cas de la comicronisation avec un agent tensio-actif tel que le laurylsulfate de sodium ou un sucrose-ester d'acide stéarique, tandis qu'elle est multipliée par 30 par rapport au mélange des poudres, dans le cas d'un cobroyage avec le désoxycholate de sodium.

D'autre part, même si la micronisation ou le broyage d'une substance en présence d'un agent tensio-actif ou d'un sucre peut augmenter sa solubilité, ces paramètres ne sont pas toujours suffisants. Par exemple, la biodisponibilité de la progestérone micronisée n'est pas satisfaisante et doit être améliorée, par exemple par dispersion dans de la cire de carnauba. Une telle technique est décrite dans la demande WO-89.02742.

Ainsi, il apparaît que les propriétés d'une substance traitée par micronisation ou broyage, en particulier sa solubilité et sa biodisponibilité, ne sont pas prévisibles, des résultats contradictoires pouvant être obtenus. De plus, une même formulation galénique peut procurer de bons résultats avec une substance et aboutir à un résultat contraire avec une autre substance.

La présente invention a pour objet une nouvelle composition pharmaceutique stable à base de diacérhéine pour administration par voie orale, rectale ou cutanée (percutanée ou transdermique), présentant une biodisponibilité améliorée, et plus particulièrement destinée au traitement des maladies inflammatoires.

La composition pharmaceutique suivant la présente invention se distingue en ce qu'elle comprend de la rhéine ou de la diacérhéine, ou un de leurs sels ou esters pharmaceutiquement acceptables, et du laurylsulfate de sodium, comicronisés, dans un rapport en poids compris entre 3:1 et 30:1.

La forme pharmaceutique utilisable dans la présente invention est adaptée à toute voie d'administration impliquant le passage à travers un épithélium, c'est-à-dire plus particulièrement les formes orale, rectale ou cutanée (percutanée ou transdermique).

Le sel ou ester pharmaceutiquement acceptable de rhéine peut être choisi parmi les esters du rhéinate de sodium ou de potassium. La diacérhéine elle-même peut être considérée comme un cas particulier, c'est-à-dire un sel de rhéine (diacétate) et un acide. Dans tout ce qui suit, la dénomination "rhéine" désigne aussi bien les acides eux-mêmes, c'est-à-dire la rhéine et la diacérhéine, que les sels ou esters.

Suivant une forme préférentielle de réalisation de l'invention, le rapport en poids de la rhéine au laurylsulfate de sodium est compris entre 6:1 et 15:1.

L'invention a encore pour objet un procédé de préparation d'une composition pharmaceutique à base de rhéine administrable par voie orale, rectale ou cutanée (percutanée ou transdermique), et présentant une biodisponibilité améliorée, consistant à comicroniser de la rhéine et du laurylsulfate de sodium, dans un rapport en poids compris entre 3:1 et 30:1, et de préférence entre 6:1 et 15:1.

La micronisation est effectuée de préférence dans un microniseur à jet gazeux, et plus particulièrement un microniseur à jet d'air ou à jet d'azote, de manière à obtenir une poudre dont la dimension moyenne des particules est inférieure à 50 µm, et de préférence inférieure à 20 µm, avec une distribution granulométrique homogène.

Le mélange de poudres comicronisées comme indiqué ci-dessus peut être mis sous forme pharmaceutique de capsule, de gélule, de crème, de pommade, de suppositoire, ou de comprimé, contenant en outre des excipients usuels. Dans le cas de formes administrables par voie orale, on peut utiliser par exemple des diluants tels que le lactose, la cellulose et des sucres, des lubrifiants tels que le stéarate de magnésium et l'acide stéarique, des délitants tels que le glycollate d'amidon-et la carboxyméthylcellulose, des anti-adhérants tels que la silice, des liants tels que la polyvidone, des composés filmogènes tels que l'hydroxypropylméthylcellulose et des laques acryliques, des plastifiants tels que le sébaçate de dibutyle et le polyéthylène glycol, des édulcorants tels que l'aspartame et le saccharinate de sodium, des opacifiants tels que le talc et le dioxyde de titane, des colorants tels que des oxydes de fer, des arômes, et des conservateurs tels que le parahydroxybenzoate de méthyle. Dans le cas des formes cutanées ou percutanées, on peut utiliser des excipients de phases grasse et aqueuse (vaseline et eau), des agents tensio-actifs et des émulsionnants tels que des esters de sorbitanne et le monostéarate de glycérol, des épaississants tels que l'alcool cétostéarylique, des gélifiants tels que des carbomères et la carboxyméthylcellulose sodique et des conservateurs tels que des parahydroxybenzoates. Dans le cas particulier de la forme transdermique, on peut utiliser des plastifiants tels que la triacétine et des polymères tels que des méthacrylates. La forme administrable par voie rectale peut comprendre des excipients lipophiles et hydrophiles tels que des glycérides, des polyoxyéthylène glycols et des suppocires®. La quantité totale d'excipients dans la composition varie généralement suivant la forme pharmaceutique utilisée, et peut représenter entre 50 et 99% environ du poids total de la composition.

Les améliorations apportées à la biodisponibilité de la rhéine par la composition de l'invention par comparaison avec les formes pharmaceutiques classiques, permettent de réduire la teneur en principe actif dans le médicament. Ainsi, dans le cas d'une formulation en gélule pour administration orale, ou d'une formulation pour administration par voie rectale, la composition suivant l'invention contient généralement entre 20 mg et 50 mg de principe actif par dose unitaire.

L'invention a enfin pour objet l'utilisation de la rhéine et du laurylsulfate de sodium comicronisés, pour la préparation d'un médicament pour le traitement des maladies inflammatoires, et en particulier de l'arthrose.

On a observé au cours des essais réalisés avec la composition de l'invention que la comicronisation du laurylsulfate de sodium avec la rhéine n'entraîne aucune dégradation de cette dernière, même dans le cas de la diacérhéine, c'est-à-dire sous forme d'un ester, qui est un produit sensible aux bases, tandis que les essais de micronisation avec un autre agent tensio-actif tel que le désoxycholate de sodium montrent une dégradation du principe actif d'autant plus importante que la concentration en agent tensio-actif est plus grande.

D'autre part, des mesures de vitesse de dissolution avec plusieurs agents tensio-actifs (rapport en poids agent tensio-actif / principe actif = 1:4 ; broyage au pilon ; temps de broyage 4 minutes) ont donné les résultats indiqués au tableau suivant (T étant le temps, exprimé en minutes, nécessaire pour dissoudre 100% du principe actif) :

| Agent tensio-actif | T |
|---|---|
| Laurylsulfate de sodium | 2 |
| Sucrose ester HLB15 * | 12 |
| Désoxycholate de sodium | 20 |

| | |
|---|---|
| * HLB = hydrophylic lipophylic balance (équilibre hydrophile - lipophile), système de classification des agents tensio-actifs mis au point par Griffin. La valeur HLB varie de 0,5 à 20, correspondant à des caractères lipophile et hydrophile, respectivement. | |

Ces résultats, en particulier l'amélioration de la vitesse de dissolution avec le laurylsulfate de sodium, sont inattendus compte tenu de ceux décrits par Otsuda et al. (précité) pour les mêmes agents tensio-actifs comicronisés avec la phénitoïne.

Les essais comparatifs de vitesse de dissolution in vitro, réalisés avec des compositions conformes à l'invention, d'une part, et des compositions contenant le principe actif non micronisé associé à un agent tensio-actif, micronisé ou non, d'autre part, ont procuré les résultats suivants :

| | 20 min. | 30 min. | 90 min. |
|---|---|---|---|
| Diacérhéine non micronisée + tensio-actif non micronisé | 60 % | | 80 % |
| Diacérhéine non micronisée + tensio-actif micronisé | 60 % | | 80 % |
| Composition de l'invention | 95 % | 100 % | 100 % |

On voit que la vitesse de dissolution du principe actif est nettement améliorée dans la composition conforme à l'invention.

Des études de bioéquivalence chez 10 volontaires sains, ont été effectuées en double aveugle, avec une table de randomisation, entre des gélules dosées à 50 mg de diacérhéine d'une forme commercialisée et des gélules dosées à 50 mg de diacérhéine et 5,6 mg de laurylsulfate de sodium, comicronisés, suivant l'invention.

Les résultats obtenus ont permis de montrer que l'amélioration de l'absorption liée à la comicronisation de la diacérhéine comicronisée avec l'agent tensio-actif est de l'ordre de 30% par rapport au produit princeps.

Ces résultats ont été confirmés par une deuxième étude de bioéquivalence en double aveugle chez 22 volontaires sains, pour laquelle la formulation princeps de 50 mg de diacérhéine s'est révélée comparable à une formulation conforme à la présente invention, contenant 35 mg de diacérhéine et 3,9 mg de laurylsulfate de sodium, comicronisée, en ce qui concerne les caractéristiques pharmacocinétiques représentées par les valeurs de Cmax (concentration plasmatique), Tmax (temps nécessaire pour parvenir à Cmax) et de la SSC (surface sous la courbe).

De plus, les essais effectués ont montré que la composition suivant l'invention présente une excellente stabilité dans le temps.

Les exemples de formulation ci-après sont donnés à titre non limitatif afin d'illustrer l'invention.

### Exemple 1

Dans un mélangeur cubique, on introduit successivement 10 kg de diacérhéine et 1 kg de laurylsulfate de sodium solide, et on mélange jusqu'à obtenir une poudre homogène.

Le mélange de poudres ainsi obtenu est comicronisé dans un broyeur à jet de gaz. On obtient ainsi une poudre dont le profil granulométrique, déterminé par granulométrie laser, est de forme gaussienne unimodale, contenant des particules de dimension moyenne inférieure à 10 µm.

On ajoute à ce mélange les excipients indiqués ci-dessous de manière à obtenir la composition par gélule n° 1 suivante :

| | |
|---|---|
| diacérhéine | 17,5 mg |
| laurylsulfate de sodium | 1,75 mg |
| cellulose | 267,5 mg |
| stéarate de magnésium | 4,5 mg |

### Exemple 2

On procède comme dans l'Exemple 1, et on introduit 10 kg de diacérhéine et 0,75 kg de laurylsulfate de sodium, successivement, dans un mélangeur cubique approprié.

Le mélange est comicronisé sur broyeur à jet de gaz, comme dans l'exemple 1, et on obtient ainsi une poudre dont le profil granulométrique, déterminé par granulométrie laser, est de forme gaussienne unimodale, contenant des particules de dimension moyenne inférieure à 10 µm.

On ajoute à ce mélange les excipients indiqués ci-dessous de manière à obtenir la composition par gélule n° 1 suivante :

| | |
|---|---|
| diacérhéine | 40 mg |
| laurylsulfate de sodium | 3 mg |
| lactose | 230 mg |
| amidon | 20 mg |
| talc | 2 mg |
| stéarate de magnésium | 10 mg |

## Revendications

1. Composition pharmaceutique à base de rhéine ou de diacérhéine pour administration par voie orale, rectale ou cutanée (percutanée ou transdermique), présentant une biodisponibilité améliorée, **caractérisée en ce qu'**elle comprend de la rhéine ou de la diacérhéine, ou un de leurs sels ou esters pharmaceutiquement acceptables, et du laurylsulfate de sodium, comicronisés, dans un rapport en poids compris entre 3:1 et 30:1.

2. Composition selon la revendication 1, **caractérisée en ce que** le rapport en poids de la rhéine ou de la diacérhéine au laurylsulfate de sodium est compris entre 6:1 et 15:1.

3. Composition selon l'une quelconque des revendications précédentes, **caractérisée en ce que** la taille moyenne des particules de rhéine ou de diacérhéine et de laurylsulfate de sodium est inférieure à 20 µm.

4. Composition selon l'une quelconque des revendications précédentes, administrable par voie orale ou rectale, **caractérisée en ce qu'**elle contient de 20 mg à 50 mg de rhéine ou de diacérhéine par dose unitaire.

5. Procédé de préparation d'une composition pharmaceutique à base de rhéine ou de diacérhéine à biodisponibilité améliorée, par micronisation du principe actif, **caractérisé en ce qu'**on effectue une comicronisation de la rhéine ou de la diacérhéine, ou d'un de leurs sels ou esters pharmaceutiquement acceptables, et du laurylsulfate de sodium dans un rapport en poids compris entre 3:1 et 30:1.

6. Procédé selon la revendication 5, **caractérisé en ce que** la micronisation est effectuée dans un microniseur à jet gazeux.

7. Utilisation de la rhéine ou de la diacérhéine et du laurylsulfate de sodium comicronisés, pour la préparation d'un médicament pour le traitement des maladies inflammatoires, et en particulier de l'arthrose.

## Claims

1. Pharmaceutical composition based on rhein or diacerein for administration by oral, rectal or cutaneous (percutaneous or transdermal) route, having improved bioavailability, **characterized in that** it comprises rhein or diacerein, or one of their pharmaceutically acceptable salts or esters, and sodium lauryl sulfate, comicronized, in a weight ratio comprised between 3:1 and 10:1.

2. Composition according to claim 1, **characterized in that** the weight ratio of rhein or diacerein to sodium lauryl sulfate is comprised between 6:1 and 15:1.

3. Composition according to any one of the preceding claims, **characterized in that** the mean size of the particles of rhein or diacerein and sodium lauryl sulfate is less than 20 µm.

4. Composition according to any one of the preceding claims, which can be administered by the oral or rectal route, **characterized in that** it contains from 20 mg to 50 mg of rhein or diacerein per unit dose.

5. Process for the preparation of a pharmaceutical composition based on rhein or diacerein with improved bioavailability, by micronization of the active ingredient, **characterized in that** comicronization of rhein or diacerein or of one of their pharmaceutically acceptable salts or esters, and of sodium lauryl sulfate is carried out in a weight ratio of between 3:1 and 30:1.

6. Process according to claim 5, **characterized in that** the micronization is carried out in a gaseous jet micronizer.

7. Use of comicronized rhein, or diacerein, and sodium lauryl sulfate, for the preparation of a medicament for the treatment of inflammatory diseases, and in particular osteoarthritis.

## Patentansprüche

1. Pharmazeutische Zusammensetzung auf Basis von Rhein oder Diacerhein zur Verabreichung auf oralem, rektalem oder kutanem (perkutanem oder transdermalem) Weg, die einer verbesserte biologische Verfügbarkeit aufweist, **dadurch gekennzeichnet, dass** sie Rhein oder Diacerhein oder eines/einen ihrer pharmazeutisch annehmbaren Salze oder Ester sowie Natriumlaurylsulfat, die gemeinsam mikrozerkleinert wurden, in einem Gewichtsverhältnis zwischen 3:1 und 30:1 enthält.

2. Zusammensetzung nach Anspruch 1, **dadurch gekennzeichnet, dass** das Gewichtsverhältnis zwischen Rhein oder Diacerhein und Natriumlaurylsulfat zwischen 6:1 und 15:1 liegt.

3. Zusammensetzung nach einem der vorangegangenen Ansprüche, **dadurch gekennzeichnet, dass** die mittlere Teilchengröße von Rhein oder Diacerhein und Natriumlaurylsulfat unter 20 µm liegt.

4. Zusammensetzung nach einem der vorangegangenen Ansprüche, die auf oralem oder rektalem Weg verabreichbar ist, **dadurch gekennzeichnet, dass** sie 20 mg bis 50 mg Rhein oder Diacerhein pro Dosiseinheit enthält.

5. Verfahren zur Herstellung einer pharmazeutischen Zusammensetzung auf Basis von Rhein oder Diacerhein mit verbesserter biologischer Verfügbarkeit durch Mikrozerkleinerung des Wirkbestandteils, **dadurch gekennzeichnet, dass** eine gleichzeitige Mikrozerkleinerung des Rheins oder des Diacerheins oder eines ihrer pharmazeutisch annehmbaren Salze oder Ester und von Natriumlaurylsulfat in einem Gewichtsverhältnis zwischen 3:1 und 30:1 durchgeführt wird.

6. Verfahren nach Anspruch 5, **dadurch gekennzeichnet, dass** die Mikrozerkleinerung in einem Gasstrahl-Mikrozerkleinerer durchgeführt wird.

7. Verwendung von Rhein oder Diacerhein und Natriumlaurylsulfat in gleichzeitig mikrozerkleinertem Zustand zur Herstellung eines Medikaments zur Behandlung von Entzündungskrankheiten, insbesondere Arthrose.
